# EUROPEAN PATENT SPECIFICATION

(11) **EP 3 791 871 B1**
(45) Date of publication and mention of the grant of the patent: **28.08.2024**
(21) Application number: 19869799.7
(22) Date of filing: 04.10.2019
(51) Int. Cl.: A61K 31/343, A23L 33/105, A61K 36/13, A61P 1/04, A61P 1/16, A61P 3/00, A61P 3/04, A61P 3/06, A61P 35/00

(54) **COMPOSITION COMPRISING GNETIN C FOR PREVENTING OR TREATING STEATOHEPATITIS BY IMPROVING INTESTINAL FLORA**
GNETIN C ENTHALTENDE ZUSAMMENSETZUNG ZUR VORBEUGUNG ODER BEHANDLUNG VON STEATOHEPATITIS DURCH VERBESSERUNG DER DARMFLORA
COMPOSITION COMPRENANT DE LA GNETINE C POUR PREVENIR OU TRAITER LA STÉATOHÉPATITE PAR AMÉLIORATION DE LA FLORE INTESTINALE

(30) Priority: 04.10.2018 JP 2018199562
(43) Date of publication of application: 17.03.2021
(73) Proprietor: MS Health Science Labo. Co., Ltd., Kanagawa 252-0816 (JP); Care-Medics Co., Ltd., Tokyo 113-0034 (JP)
(72) Inventor: WATANABE, Mitsuhiro, Yokohama-shi, Kanagawa 241-0021 (JP); HOSODA, Shotaro, Fukui-shi, Fukui 910-0854 (JP)
(74) Representative: dompatent von Kreisler Selting Werner - Partnerschaft von Patent- und Rechtsanwälten mbB
(86) International application number: PCT/JP2019/039307
(87) International publication number: WO 2020/071541

(56) References cited:
- EP-A1- 1 790 239
- CN-A- 108 236 607
- JP-A- 2006 273 834
- JP-A- 2009 013 123
- JP-A- 2009 249 320
- JP-A- 2010 184 886
- JP-A- 2014 101 341
- JP-A- 2017 081 891
- JP-A- 2019 077 657
- JP-B1- 6 362 237
- JP-B2- S6 362 237
- YEH CHAO-BIN ET AL: "The Antimetastatic Effects of Resveratrol on Hepatocellular Carcinoma through the Downregulation of a Metastasis-Associated Protease by SP-1 Modulation", PLOS ONE, vol. 8, no. 2, 20 February 2013 (2013-02-20), pages e56661, XP055787244, Retrieved from the Internet <URL:https://storage.googleapis.com/plos-corpus-prod/10.1371/journal.pone.0056661/1/pone.0056661.pdf?X-Goog-Algorithm=GOOG4-RSA-SHA256&X-Goog-Credential=wombat-sa@plos-prod.iam.gserviceaccount.com/20210318/auto/storage/goog4_request&X-Goog-Date=20210318T094038Z&X-Goog-Expires=3600&X-Goog-SignedHeaders=ho> DOI: 10.1371/journal.pone.0056661
- DATABASE BIOSIS [online] BIOSCIENCES INFORMATION SERVICE, PHILADELPHIA, PA, US; February 2001 (2001-02-01), HOU YAN-NING ET AL: "Effect of stilbene polymer (Gn-3) on experimental liver injuries in mice", XP002802446, Database accession no. PREV200100228245
- HOU YAN-NING ET AL: "Effect of stilbene polymer (Gn-3) on experimental liver injuries in mice", YAOXUE XUEBAO, vol. 36, no. 2, February 2001 (2001-02-01), pages 81 - 83, ISSN: 0513-4870
- CHONGYANG ZHANG ET AL: "High-dose resveratrol supplementation in obese men: an investigator-initiated, randomized, placebo-controlled clinical trial of substrate metabolism, insulin sensitivity, and body composition", PLOS ONE, vol. 62, no. 8, 25 August 2016 (2016-08-25), pages 1186, XP055332701, DOI: 10.1371/journal.pone.0161792
- KATAOKA SAORI ET AL: "Melinjo (Gnetum gnemon) extract intake during lactation stimulates hepatic AMP-activated protein kinase in offspring of excessive fructose-fed pregnant rats", REPRODUCTIVE BIOLOGY, vol. 16, no. 2, 1 June 2016 (2016-06-01), Poland, pages 165 - 173, XP055787181, ISSN: 1642-431X, DOI: 10.1016/j.repbio.2016.01.002
- DATABASE BIOSIS [online] BIOSCIENCES INFORMATION SERVICE, PHILADELPHIA, PA, US; February 2001 (2001-02-01), HOU YAN-NING ET AL: "Effect of stilbene polymer (Gn-3) on experimental liver injuries in mice", XP002802446, Database accession no. PREV200100228245
- YAOXUE XUEBAO, vol. 36, no. 2, February 2001 (2001-02-01), pages 81 - 83, ISSN: 0513-4870
- BISHAYEE ANUPAM ET AL: "Resveratrol and liver disease: from bench to bedside and community : Resveratrol and liver disease", LIVER INTERNATIONAL, vol. 30, no. 8, 14 June 2010 (2010-06-14), GB, pages 1103 - 1114, XP055787594, ISSN: 1478-3223, DOI: 10.1111/j.1478-3231.2010.02295.x
- FAGHIHZADEH FOROUZAN ET AL: "Resveratrol and liver: A systematic review", JOURNAL OF RESEARCH IN MEDICAL SCIENCES, vol. 20, no. 8, 1 January 2015 (2015-01-01), INDIA, pages 797, XP055787712, ISSN: 1735-1995, DOI: 10.4103/1735-1995.168405
- YONESHIRO TAKESHI; KAEDE RYUJI; NAGAYA KAZUKI; SAITO MANAMI; AOYAMA JULIA; ELFEKY MOHAMED; OKAMATSU-OGURA YUKO; KIMURA KAZUHIRO; T: "Melinjo (Gnetum gnemon L.) seed extract induces uncoupling protein 1 expression in brown fat and protects mice against diet-induced obesity, inflammation, and insulin resistance", NUTRITION RESEARCH, vol. 58, 3 July 2018 (2018-07-03), pages 17 - 25, XP085506666, ISSN: 0271-5317, DOI: 10.1016/j.nutres.2018.06.012
- NARAYANAN K NARAYANAN; KAZUHIRO KUNIMASA; YUKIO YAMORI; MARI MORI; HIDEKI MORI; KAZUKI NAKAMURA; GEORGE MILLER; UPENDER MANNE; AMI: "Antitumor activity of melinjo (Gnetum gnemon L. ) seed extract in human and murine tumor models in vitro and in a colon-26 tumor-bearing mouse model in vivo", CANCER MEDICINE, vol. 4, no. 11, 1 November 2015 (2015-11-01), pages 1767 - 1780, XP055545730, ISSN: 2045-7634, DOI: 10.1002/cam4.520
- TAKASHINO, YOKO : "P03: Medicinal efficacy of gnetin C (resveratolol dimer)", PROGRAMS AND ABSTRACTS OF THE 16TH SCIENTIFIC MEETING OF THE JAPANESE SOCIETY OF ANTI-AGING MEDICINE, 1 January 2016 (2016-01-01), pages 200, XP009524806
- KATO EISHIN: "The biological action of melinjo ingredients", JOURNAL OF JAPAN HEALTH MEDICINE ASSOCIATION, vol. 16, no. 3, 30 November 2006 (2006-11-30), pages 74 - 75, XP009524765, ISSN: 1343-0025, DOI: 10.20685/kenkouigaku.16.3_74
- YAMAGUCHI, TOSHIYUKI ET AL.: " A paradigm shift in cancer treatment brought by immune checkpoints (PD-1 / PD-L1)", RINSHO GEKA = JOURNAL OF CLINICAL SURGERY, vol. 72, no. 7, 2017, pages 876 - 880, XP009524764, ISSN: 0386-9857

## Description

### Technical Field

The present invention relates to a composition for improving intestinal flora that contains, as active ingredients, gnetin C and/or a glycoside thereof having an effect of improving intestinal flora by proliferating Akkermansia muciniphila.

### Background Art

Intestinal flora is formed by a huge number of microorganisms in the intestine of human and, in the recent years, it becomes clear that changes in the taxonomic composition of this bacterial flora are associated with diseases. The intestinal flora, which is deeply associated in health and diseases, is reported to be involved in intestinal disorders such as inflammatory bowel disease, irritable bowel syndrome, and colorectal cancer, lifestyle disease, autoimmune disease, autism, and the like. Changes in the intestinal flora caused by aging reportedly affect health of the host (Non Patent Literature 1).

Further, there is a theory that fatty liver caused by fat deposition in the liver cells advances to liver diseases (such as nonalcoholic fatty liver disease and nonalcoholic steatohepatitis) by further occurrence of inflammation and progression of fibrosis. In this theory, intestinal microbiota is taken into account as a cause of the fatty liver (Non Patent Literature 2). Further, it is reported that blocking deoxycholic acid production or reducing intestinal bacteria efficiently prevents hepatocellular carcinoma development in obese mice (Non Patent Literature 3). It is also known that lithocholic acid produced by intestinal bacteria as a secondary bile acid is involved in the occurrence of colorectal cancer (Non Patent Literature 4). There is a report that the ratio in the intestinal flora of Akkermansia, which exhibits an anti-inflammatory effect and an insulin resistance effect in adipose tissues, is low in males having high risk of developing diabetes (Non Patent Literature 5). It is reported that cancer patients who fail to respond to immune checkpoint inhibitors restore the efficacy of the inhibitors by oral supplementation with Akkermansia after fecal microbiota transplant (Non Patent Literature 6). Further, it is reported that intestinal bacteria such as Akkermansia are reduced in ulcerative colitis (Non Patent Literature 7). Patent Literature 1 discloses that pentameric or higher polymeric proanthocyanidins derived from plants promote proliferation of Akkermansia sp. bacteria.

It is disclosed that gnetin C and a glycoside thereof, which are low-molecular weight compounds contained in endosperms of melinjo seeds, are major components of melinjo extract obtained by extraction using a hydrous alcohol or the like (Patent Literature 2 and Non Patent Literature 8). For example, EP1790239, JP6362237, JP2010184886 and JPS6362237 discloses a Melinjo/Genetum gnemon (seed) extract containing gnetin C. JP2017081891 discloses a composition comprising a melange extract containing stilbenes of melinjo seeds as a useful ingredient, wherein the stilbenes include the dimers gnetic C, gnemonoside A, gnemonoside C and gnemonoside D. Further, the extract and its ingredients have various uses including medical uses. For example, JP2010184886 discloses a Melinjo extract containing gnetic C, gnemonoside A, gnemonoside C and gnemonoside D and its use in the prevention of visceral fat obesity by reducing blood cholesterol and blood triglycerides. In addition, JP2009249320 discloses a Genetum/melinjo extract for use in improving diabetes, increasing leptin and/or decreasing glucose in blood. JP2009013123 discloses products containing Gnetum extract which have polysaccharide hydrolase inhibitory effect, triglycerides lowering action and/or a hypoglycemic effect. Reproductive Biology, 16, 2, 165-173 (2016) discloses the effects of Melinjo/Gnetum gnemon extract intake during lactation on the expression and phosphorylation of adenosine monophosphate-activated protein kinase in the liver of offspring from excessive fructose-fed pregnant dams. JP2014101341 discloses a resveratrol dimer extracted from Melinjo which suppresses secretion of ApoB100 from a liver. JP2019077657 discloses the use of a resveratrol dimer selected from the group consisting of gnetin C, gnetin L, gnemonoside A, gnemonside C and gnemonside D as simultaneous inhibitors of aberrant activation of rapamycin (mTOR) signaling pathway and mitogen-activated protein kinase signaling pathway for the prophylaxis or treatment of cancer. JP2006273834 discloses the use of resveratrol as AMP-activated protein kinase ACTIVATOR, lipid-metabolism activator, obesity inhibitor, diabetes presentation improving agent, liver enlargement inhibitor and fatty liver inhibitor. CN108236607 discloses the stilbene dimer Gnetulin isolated from Gnetum in the treatment of liver diseases, viral hepatitis, chemical or drug-induced liver damage. Database Biosis, 36, 2, 81-83(2001) discloses the protective effect of Gn-3, which is a stilbene polymer isolated from Gnetum parvifolium, against liver injury induced CCL4, N-acetyl-p-aminophenol and Bacillus Calmette-Guerin plus bacterial lipopolysaccharide in mice. Liver International, 30, 8, 1103-1114(2010) and Journal of Research in Medical Sciences, 20, 8, 797(2015) disclose the therapeutic uses of resveratrol in the treatment of liver diseases. Nutrition research, 58, 17-25(2018) discloses the anti-obesity effects of Melinjo seed extract in high fat diet fed mice. It further discloses that dietary Melinjo seed extract protected against HFD-induced fat accumulation and fatty liver. Cancer medicine, 4, 11, 1767-1780(2015) discloses the antitumor activity of Melinjo seed extract and its active ingredient gnetin C using human and murine tumor cell culture models in vitro. Programs and abstracts of the 16th Scientific Meeting of the Japanese Society of Anti-Aging Medicine, page 200(2016-01-01) discloses a study investigated the anti-obesity effect of using Melinjo seed extract containing gnetin C in high fat diet-fed mice, which suggested that the extract increases energy consumption and corrects haptic insulin resistance by activating SIRT1 and SIRT3 and controlling bile acid metabolism. Journal of Japan Health Medicine Association, 16, 3, 74-75(2006) discloses the antibacterial effect of melinjo extract powder containing gnetin C. However, it was not known that gnetin C and the glycoside thereof had an effect of improving the intestinal flora by proliferating Akkermansia.

### Citation List

### Patent Literature

Patent Literature 1: JP 2018-8911 A
Patent Literature 2: WO 2006/030771 A

### Non Patent Literature

Non Patent Literature 1: Japanese journal of geriatrics, 53, 318 (2016)
Non Patent Literature 2: Modern media, 60, 12, 356-368 (2014)
Non Patent Literature 3: S. Yoshimoto, et al. Nature, 499, 97 (2013)
Non Patent Literature 4: V. Kozoni, et al. Carcinogenesis, 21, 999 (2000)
Non Patent Literature 5: A. Everand, et al. Pro. Natl. Acad. Sci. USA. 110, 22 (2013)
Non Patent Literature 6: B. Rouly, et al. Science, 359, 356 (2018)
Non Patent Literature 7: L. Bajer, et al. World J. Gastroenterology, 23, 4548 (2017)
Non Patent Literature 8: J. Agric. Food Chem. 57, 2544 (2009)

### Summary of Invention

The invention is defined in the claims

### Technical Problem

It is an object of the present disclosure to provide a composition for improving intestinal flora by utilizing a component obtained from a seed of melinjo which is a safe plant used as food.

Further, it is another object of the present disclosure to provide a preventing and/or improving agent of a disease by improving the intestinal flora, an agent for improving an effect of an immune checkpoint inhibitor, and a bile acid metabolic function regulator.

### Solution to Problem

As a result of intensive studies for solving the aforementioned problems, the present inventors have found that a mouse fed with a composition containing gnetin C and a glycoside thereof as active ingredients exhibits a proliferation effect of Akkermansia muciniphila (hereinafter simply referred to as Akkermansia) in intestinal flora, thereby completing the present invention.

That is, the present invention that solves the aforementioned problems is defined in the appended claims 1 and 2.

The intestinal flora improving agent of the present invention can promote proliferation of Akkermansia in the intestinal flora or increase an abundance ratio of Akkermansia in the intestinal flora.

In a preferred embodiment of the present invention, the glycoside is one or two or more selected from gnemonoside A, gnemonoside C, or gnemonoside D.

These glycosides are hydrolyzed into gnetin C in the intestinal tract, thereby providing the same effect as in the case where gnetin C is directly ingested.

In a preferred embodiment described herein, the composition for improving intestinal flora is melinjo extract.

In a preferred embodiment described herein, the composition for improving intestinal flora has an effect of increasing an abundance ratio of Akkermansia in the intestinal flora.

Further, the present disclosure provides a preventing agent and/or an improving agent of a liver function disease, which contain the aforementioned composition for improving intestinal flora.

The aforementioned composition for improving intestinal flora has an effect of preventing or improving the liver function disease by reducing fat deposition in the liver and regulating the metabolism of a bile acid.

The aforementioned liver function disease is nonalcoholic steatohepatitis.

The aforementioned composition for improving intestinal flora has an effect of preventing or improving liver cancer by reducing fat deposition in the liver and lowering amounts of deoxycholic acid and lithocholic acid, which are carcinogenic secondary bile acids.

Described is a preventing agent and/or an improving agent of colorectal cancer, which contain the aforementioned composition for improving intestinal flora.

The aforementioned composition for improving intestinal flora has an effect of preventing or improving colorectal cancer by lowering amounts of deoxycholic acid and lithocholic acid, which are carcinogenic secondary bile acids.

Further, described is an agent for improving an effect of an immune checkpoint inhibitor, which contains the aforementioned composition for improving intestinal flora.

The aforementioned composition for improving intestinal flora has an effect of improving an effect of the immune checkpoint inhibitor by promoting proliferation of Akkermansia or increasing the abundance ratio thereof.

Further, described is an agent for treating an inflammatory bowel disease, which contains the aforementioned composition for improving intestinal flora.

The aforementioned composition for improving intestinal flora can reduce fat deposition and indirectly reduce inflammation caused by the deposited fat.

Further, described is a bile acid metabolism regulator, which contains the aforementioned composition for improving intestinal flora.

The aforementioned composition for improving intestinal flora has an effect of regulating the bile acid metabolism.

Further, described are food and drink, which contain the aforementioned composition for improving intestinal flora.

The aforementioned composition for improving intestinal flora contains, as active ingredients, gnetin C and/or the glycoside thereof, which are components included in melinjo extract safe as food, and is thus suitably ingested as food and drink.

### Advantageous Effects of Invention

The composition for improving intestinal flora uses melinjo, which is safe food frequently eaten in Indonesia, and the extract of melinjo, and gnetin C and the glycoside thereof, which are components included in melinjo, have an effect of promoting proliferation of Akkermansia in the intestinal flora.

The composition for improving intestinal flora having this effect can be used for preventing and treating nonalcoholic steatohepatitis (NASH).

### Brief Description of Drawings

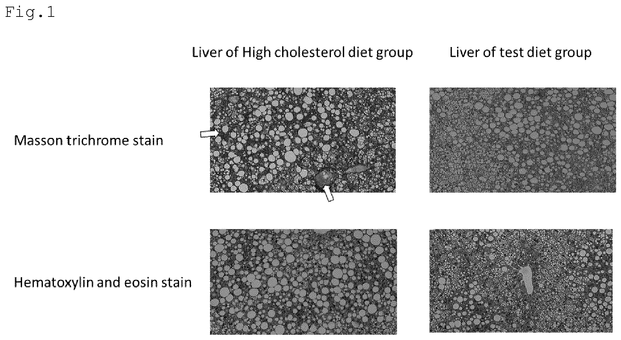

Fig. 1 shows micrographs of liver tissues of a mouse fed with a high-fat diet and a mouse fed with a test diet in Test example 3.

### Description of Embodiments

Improving intestinal flora by a composition for improving intestinal flora means increasing an abundance ratio of Akkermansia muciniphila in the intestinal flora. Akkermansia belonging to the genus Akkermansia of the phylum Verrucomicrobia is a Gram-negative, strictly anaerobic, non-motile, and non-spore-forming bacterium. Akkermansia is able to use mucin as carbon and nitrogen sources and colonize the gastrointestinal tracts of animal species. Akkermansia has an anti-inflammatory effect.

The composition for improving intestinal flora uses gnetin C and/or a glycoside thereof as active ingredients.

Gnetin C is a compound represented by the following formula (1). Gnemonoside C, gnemonoside D, and gnemonoside A can be mentioned as the glycoside of gnetin C.

Gnemonoside C is a compound in which the hydroxy group of the hydroxyphenyl group replaced to the furan ring is replaced to a glycosyl group in the following formula (1), gnemonoside D is a compound in which the hydroxy group of the hydroxystyryl group is replaced to a glycosyl group, and gnemonoside A is a compound in which both of the hydroxy groups of the hydroxyphenyl group and the hydroxystyryl group are replaced to glycosyl groups.

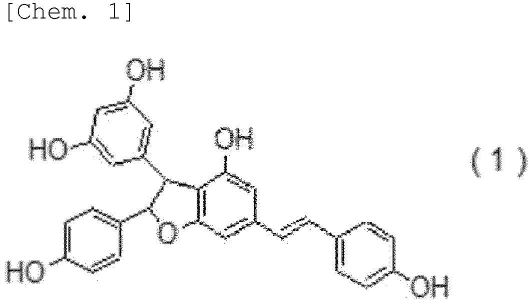

Gnetin C and/or the glycoside thereof are main components of a melinjo seed endosperm. Thus, gnetin C and the glycoside thereof can be obtained by preparing melinjo extract from melinjo by extraction and subjecting the melinjo extract to separation and purification. For example, the melinjo extract can be obtained by the extraction method described in Patent Literature 2. This extract is subjected to the method described in Non Patent Literature 6 using an octadecyl silica gel (ODS) column and a silica gel column, and separation and purification using a polystyrene resin column to obtain gnetin C and the glycoside thereof, respectively.

Specifically, the melinjo extract can be obtained by using one or two or more selected from a pericarp, a seed coat, a thin skin, or an endosperm of melinjo as an extraction raw material.

As an extracting agent, water and/or an organic solvent can be used, and a water-based extracting agent prepared as a mixed solvent of water and a polar organic solvent is preferably used.

Examples of the organic solvent include 1) alcohols (including diols and triols) such as methanol, ethanol, 1-propanol, 2-propanol, 1-butanol, ethylene glycol, propylene glycol, and glycerin, 2) ethers (including cyclic ethers) such as diethyl ether, cellosolve, dioxane, and tetrahydrofuran, 3) esters such as methyl acetate, ethyl acetate, and cellosolve acetate, 4) ketones such as acetone and organic acids being liquid at normal temperature such as acetic acid, glacial acetic acid, and propionic acid, 5) amines such as ethylenediamine, pyridine, and monomethanolamine, 6) hydrocarbons (including aliphatic, alicyclic, and aromatic) such as hexane, cyclohexane, heptane, benzene, toluene, and xylene, and 7) halocarbons such as methylene chloride, chloroform, carbon tetrachloride, 1,2-dichloroethane, dichloroethylene, and trichloroethylene.

Of these described above, lower alcohols miscible with water at any ratio such as methanol, ethanol, propanol, and butanol, ethers such as diethyl ether, esters such as methyl acetate and ethyl acetate, ketones such as acetone, and organic acids being liquid at normal temperature such as acetic acid, glacial acetic acid, and propionic acid can be preferably used as the polar organic solvent used in the water-based extracting agent. Of these, in a case where the composition for improving intestinal flora of the present invention is prepared as an oral agent, the solvent having a high allowable residual concentration (e.g., ethanol) is desirably used without necessity of subsequent purification.

The composition for improving intestinal flora promoting proliferation of Akkermansia can be used as a preventing agent and a treating agent which can be administered and ingested for preventing and/or treating liver function disease, inflammatory bowel disease, cancer, lifestyle disease, and the like.

The intestinal flora composition can be used in the form of food for specified health uses, food with functional claims, health food and drink, and the like.

The composition for improving intestinal flora can be prepared as a paste product, a solid product, and a powdery product by including a compounding agent using a general method for facilitating administration and ingestion, and then processed into a preparation such as a granular agent, a tablet, a capsule agent, or a drinking agent, and food and drink such as a beverage, chocolate, a cereal flour product, or a dairy product. As the compounding agent, an excipient such as an emulsifier, dextrin, starch, or a sugar, a thickening/gelling agent, a disintegrant, a lubricating agent, fat and oil, a higher alcohol, and the like can be appropriately used depending on the processing form. In the case of food and drink, a compounding agent generally used as a food raw material can be appropriately compounded.

The dosage (dose of administration and ingestion) of the composition used as an oral agent for promoting proliferation of Akkermansia varies depending upon the purpose of the administration and conditions of a person to be administered (gender, age, weight, degree of obesity, degree of total health, etc.). Typically, as a daily dosage, the composition can be administered in a range of from 10 to 1000 mg/person/day in terms of the weight of gnetin C. Further, the composition can be applied to animals in the same manner.

### Examples

Hereinafter, the present invention will be described in detail by way of Examples. However, the present invention is not limited to these Examples.

### Production example 1 (melinjo extract powder)

In accordance with the method in Patent Literature 1, a melinjo seed endosperm was immersed in a hydrous ethanol at room temperature, and an extract liquid obtained by filtration was concentrated under the reduced pressure to obtain melinjo extract. The melinjo extract was dissolved in water, and dextrin was added and dissolved therein. After that, the resulting mixture was freeze-dried to produce melinjo extract powders (Lot OMP-517061 manufactured by Hosoda SHC Co., Ltd.). The melinjo extract powders thus obtained were used in Test example. According to an HPLC analysis of the powders, the powders contained 19.9% of gnemonoside A, 4.3% of gnemonoside D, and 2.3% of gnetin C.

### Production example 2 (separation and purification of gnemonoside A and gnetin C)

In accordance with the method in Patent Literature 1, 2 kg of melinjo seed endosperms was immersed in 6 L of 60% ethanol at room temperature for 2 days, and an extract liquid obtained by filtration was concentrated under the reduced pressure to obtain 330 g of melinjo extract in a paste form. This melinjo extract in an amount of 150 g was dissolved in water, subjected to an ODS column, and eluted and separated with a hydrous methanol. The separation and purification were performed in accordance with the method in Non Patent Literature 6 to obtain 9 g of gnemonoside A in a form of amorphous powders and 11 g of gnetin C in a form of amorphous powders. Gnetin C thus obtained was subjected to a silica gel column and eluted and purified with a mixture liquid of methylene chloride and ethanol to obtain 10 g of gnetin C in a form of amorphous powders.

### (Test example 1)

Eighteen mice were divided into a normal diet group consisting of 6 mice in which the mice were fed with a normal diet (D12450B manufactured by Research Diets, Inc.), a high-fat diet group consisting of 6 mice in which the mice were fed with a high-fat diet (D12492 manufactured by Research Diets, Inc.), and a test diet group consisting of 6 mice in which the mice were fed with a test diet in which 0.5% of the melinjo extract powders produced in Production example 1 was included in the high-fat diet. The mice were fed ad libitum with these diets in an amount of 5g/mouse/day for 8 consecutive weeks and then their excrement was collected and stored after freeze drying.

After the freeze-dried excrement was crushed, 1% SDS and a phenol/chloroform/isoamyl alcohol mixed solution were added to 10 mg of the excrement, and the mixture was stirred and then subjected to centrifugal separation. To the supernatant, the phenol/chloroform/isoamyl alcohol mixed liquid was added, and the resulting mixture was mixed and further subjected to centrifugal separation.

Next, to the supernatant, 3M sodium acetate solution and cold ethanol were added, and the resulting mixture was mixed and allowed to stand at -80°C. After that, the mixture was subjected to centrifugal separation to remove ethanol.

Next, 70% ethanol was added and mixed into the precipitate. After the upper layer was removed by performing centrifugal separation, the remaining precipitate was dried.

To the dried product, 0.1 mL of Tris buffer (pH8) containing EDTA was added, and the resulting mixture was stirred and subjected to centrifugal separation. After that, 20 µL of the supernatant was stored at -30°C, and the remaining liquid was added with RNase A and incubated at 37°C overnight.

The liquid after incubation was adjusted to 200 µL by adding pure water, and 200 µL of the phenol/chloroform/isoamyl alcohol mixed solution was added to the liquid. The resulting mixture was stirred and then subjected to centrifugal separation.

Next, 20 µL of 3M sodium acetate solution (pH5.2) was added and mixed into the supernatant, followed by addition of 400 µL of cold ethanol. The resulting mixture was mixed, cooled on ice, and then subjected to centrifugal separation to separate the supernatant. To the precipitate, 500 µL of 70% ethanol was added, and the resulting mixture was mixed and subjected to centrifugal separation under cooling to remove the upper layer. After the same operation was repeated, the resulting precipitate was dried.

To the dried product, 50 µL of Tris buffer (pH8) containing EDTA was added, and the resulting mixture was stirred and subjected to measurement of DNA concentration using Nanodrop. The concentration of DNA was diluted to 10 ng/µL with Tris buffer (pH8) containing EDTA.

To 1 µL of the diluted liquid, 1.25 µL of F Primer (27 Fmod: 10 µM), 1.25 µL of R Primer (338R: 10 µM), 25 µL of 2x Gflex PCR buffer, 1 µL of Tks Gflex DNA polymerase, and 20.5 µL of pure water were mixed to prepare a sample liquid.

The sample liquid was subjected to a PCR reaction in which a cycle of 98°C, 55°C, and 68°C was repeated 20 times, and then stored at 4°C. A mixed liquid consisting of 5 µL of each sample liquid and 1 µL of 6x Loading buffer was subjected to 2% agarose gel (Tris buffer containing EDTA) electrophoresis (marker: fX174/HaeIII digest), followed by a bacteria analysis. An intestinal flora analysis was performed on the basis of this result.

The result is shown in Table 1. Note that the number in the Table indicates an average value of the mice (6 mice) in each group.

**[Table 1]**

| Phylum | Normal diet group | High-fat diet group | Test diet group |
|---|---|---|---|
| Actinobacteria | 2.70% | 1.60% | 0% |
| Bacteroides | 27.80% | 38.80% | 36.50% |
| Cyanobacteria | 0% | 0% | 0% |
| Deferribacter | 2. 00% | 2.50% | 0% |
| Firmicutes | 57.40% | 42.80% | 1.80% |
| Proteobacteria | 8.90% | 12.40% | 18.60% |
| Tenerictes | 0% | 0% | 0% |
| Verrucomicrobia | 0% | 0.20% | 41.80% |
| Others | 1.20% | 0% | 1.30% |

| | | | |
|---|---|---|---|
| (n=6) | | | |

Performing the intestinal flora analysis revealed that bacteria of the phylum Firmicutes accounted for 57.4%, bacteria of the phylum Bacteroidetes accounted for 27.8%, and bacteria of the phylum Proteobacteria accounted for 8.9% in the normal diet group.

It is revealed that bacteria of the phylum Firmicutes accounted for 42.8%, bacteria of the phylum Bacteroidetes accounted for 38.8%, and bacteria of the phylum Proteobacteria accounted for 12.4% in the high-fat diet group.

On the other hand, in the test diet group, bacteria of the phylum Verrucomicrobia, not found in the normal diet group or the high-fat diet group, accounted for 41.8%, bacteria of the phylum Firmicutes accounted for 1.8%, bacteria of the phylum Bacteroidetes accounted for 36.5%, and bacteria of the phylum Proteobacteria accounted for 18.6%. A bacterial species of the phylum Verrucomicrobia confirmed in the test diet group was Akkermansia sp. bacteria with the abundance ratio of 41.8%.

This result shows that the composition for improving intestinal flora of the present invention can promote proliferation of Akkermansia or have an effect of increasing an abundance ratio of Akkermansia in the intestinal flora.

### (Test example 2)

Eighteen mice in Test example 1 were dissected, and the liver weight was measured.

The result is shown in Table 2.

**[Table 2]**

| | Normal diet group | High-fat diet group | Test diet group |
|---|---|---|---|
| Liver weight (g) | 1. 14 | 2. 35 | 1.48 |

| | | | |
|---|---|---|---|
| (n=6) | | | |

The measurement result showed that the liver weight in the high-fat diet group was 2.1 times that in the normal diet group.

On the other hand, the liver weight in the test diet group was 1.3 times that in the normal diet group.

This result shows that fat accumulation in the liver in the test diet group in which the high-fat diet including the melinjo extract powders was fed was reduced to half or less than that in the high-fat diet group. Further, from this result, it is speculated that fat accumulation in the liver was reduced due to proliferation of Akkermansia in the intestinal flora or an increase in the abundance ratio of Akkermansia in the intestinal flora (see Non Patent Literature 2).

### (Test example 3)

Eighteen mice in Test example 1 were dissected, and a bile acid analysis in the liver and a bile acid analysis in the excrement were performed in the following manner.

To an incubation tube with deuterated bile acids as an internal standard in it, 20 mg of the liver sample of each mouse was put, and 10 µL of 8.9 M potassium hydroxide solution and 90 µL of sterile pure water were added therein. The resulting mixture was mixed and then incubated at 80°C for 20 minutes. To this mixture, 2 mL of 0.5M potassium phosphate buffer (pH7.3) was added, and the resulting mixture was mixed and then subjected to centrifugal separation to prepare a sample liquid. Each sample liquid was subjected to a liquid chromatography-mass spectrometry analysis (LCMS analysis) to obtain the amount of each bile acid. The result is shown in Table 3.

Further, to a tube in which 20 mg of each excrement sample was put, 50 µL of 8.9 M potassium hydroxide solution and 450 µL of sterile pure water were added. The resulting mixture was mixed, incubated at 80°C for 20 minutes, and then subjected to centrifugal separation to collect the supernatant. Each supernatant in an amount of 10 µL was added in the incubation tube with deuterated bile acids as an internal standard in it to prepare a sample liquid, and the sample liquid was subjected to the LCMS analysis to obtain the amount of each bile acid. The result is shown in Table 4.

**[Table 3]**

| Bile acid (µM) | Normal diet group | High-fat diet group | Test diet group |
|---|---|---|---|
| Cholic acid | 0.28 | 0.22 | 2.9 |
| Taurocholic acid | 0.98 | 0.85 | 1.54 |
| Taurodeoxycholic acid | 0.17 | 0.07 | 0 |
| Taurocheodeoxychoic acid | 0.08 | 0.07 | 0.13 |
| Tauroursodeoxycholi c acid | 0.14 | 0.07 | 0.19 |
| *β* -Muricholic acid | 0.02 | 0 | 0.03 |
| Tauro- *β* -murcholic acid | 0.08 | 0.16 | 1.31 |

| | | | |
|---|---|---|---|
| (n=6) | | | |

**[Table 4]**

| Bile acid (µM) | Normal diet group | High-fat diet group | Test diet group |
|---|---|---|---|
| Cholic acid | 0.4 | 0.88 | 10.9 |
| Taurocholic acid | 0.03 | 0.14 | 0.44 |
| Deoxycholic acid | 1.54 | 25.45 | 1.16 |
| Lithocholic acid | 0.23 | 5.06 | 0 |
| Ketritocholic acid | 0.45 | 6.18 | 0.06 |
| Hyodeoxycholic acid | 0.03 | 2.49 | 0.06 |
| *β*-Muricholic acid | 1.87 | 5.09 | 16.19 |
| Tauro-*β*-Muricholic acid | 0.08 | 0.16 | 1.31 |

| | | | |
|---|---|---|---|
| (n=6) | | | |

The result of the analysis showed that the contents of cholic acid, taurocholic acid, and tauro-β-muricholic acid in the high-fat diet group were 0.8 times, 0.9 times, and 2 times those in the normal diet group, respectively.

On the other hand, the contents of cholic acid, taurocholic acid, and tauro-β-muricholic acid in the test diet group were 10 times, 1.6 times, and 16 times those in the normal diet group, respectively, showing that the contents of all of the bile acids significantly increased.

These results show that the composition for improving intestinal flora of the present invention increases the bile acid content by changing the intestinal flora and has an effect of reducing the liver function disease such as nonalcoholic fatty liver disease (NAFLD) or nonalcoholic steatohepatitis (NASH).

The result of the bile acid analysis in the excrement showed that the contents of cholic acid, deoxycholic acid, lithocholic acid, β-muricholic acid, and tauro-β-muricholic acid in the high-fat diet group were 2.2 times, 16.5 times, 22.0 times, 2.7 times, and 2.0 times those in the normal diet group, respectively.

On the other hand, the contents of cholic acid, deoxycholic acid, lithocholic acid, β-muricholic acid, and tauro-β-muricholic acid in the test diet group were 27.3 times, 0.8 times, 0 times (lithocholic acid was not found in the test diet group), 8.7 times, and 16.4 times those in the normal diet group, respectively.

These indicate that, in the high-fat diet group, the ratios of deoxycholic acid and lithocholic acid, which are deoxygenated carcinogenic substances, significantly increase, while, in the test diet group, the contents of cholic acid, β-muricholic acid, and tauro-β-muricholic acid significantly increase and the contents of carcinogenic deoxycholic acid and lithocholic acid significantly decreases due to changes in the aforementioned intestinal flora. This result suggests that the composition for improving intestinal flora of the present invention has an effect of reducing carcinogenesis of colorectal cancer and the like by changing the intestinal flora.

### (Test example 4)

Twenty-one mice were divided into a normal diet group consisting of 7 mice, a high-fat diet group consisting of 7 mice, and a test diet group consisting of 7 mice in which 0.5% of gnetin C separated and purified in Production example 2 was included in the high-fat diet. The mice were fed ad libitum with these diets in an amount of 5g/mouse/day for 14 consecutive weeks and their weights were measured every week. The result is shown in Table 5.

**[Table 5]**

| Weight | Normal diet group | High-fat diet group | Test diet group |
|---|---|---|---|
| Start date weight (g) | 20 | 20.1 | 20 |
| 14 week later weight (g) | 28.4 | 45 | 28.6 |

| | | | |
|---|---|---|---|
| (n=7) | | | |

The weights in the normal diet group and the test diet group slowly increased over the weeks and their weights after 14 weeks were 1.42 times and 1.43 times those at the start time, respectively.

On the other hand, the weight in the high-fat diet group significantly increased over the weeks and the weight after 14 weeks was 2.24 times that at the start time.

This result shows that the composition for improving intestinal flora of the present invention has an effect of reducing fat accumulation.

### (Test example 5)

Twenty-one mice in Test example 4 were dissected, and the liver weight, the content of liver triglyceride, and the visceral fat weight were measured.

The liver and the intestinal tract membrane added with a chloroform/methanol mixed solution were crushed by a homogenizer and then subjected to centrifugal separation to collect the supernatant. The solvent was distilled off under the reduced pressure from each supernatant to obtain a residue. The residue was dissolved by adding 2-propanol and used for measuring the amount of liver triglyceride with an enzyme analysis kit, Determiner L TGII (manufactured by Kyowa Medex Co., Ltd.), and the total cholesterol amount with Cholesterol E-test Wako (Wako Pure Chemical Industries, Ltd.). The result is shown in Table 6.

**[Table 6]**

| Item | Normal diet group | High-fat diet group | Test diet group |
|---|---|---|---|
| Liver weight (g) | 0.94 | 1.42 | 0.8 |
| Liver triglyceride (mg) | 59 | 381 | 36 |
| Visceral fat (g) | 0.32 | 1.13 | 0.3 |

| | | | |
|---|---|---|---|
| (n=7) | | | |

The liver weight, the content of liver triglyceride, and the visceral fat weight in the high-fat diet group were 1.5 times, 6.5 times, and 3.5 times those in the normal diet group.

On the other hand, the liver weight, the content of liver triglyceride, and the visceral fat weight in the test diet group were 0.9 times, 0.6 times, and 0.9 times those in the normal diet group.

The liver weight, the content of liver triglyceride, and the visceral fat weight in the test diet group were about a half, one tenth, and a quarter of those in the high-fat diet group.

In the high-fat diet group, the liver weight, the content of liver triglyceride, and the visceral fat weight significantly increased, while, in the test diet group, all of these parameters were lower than those in the normal diet group.

This result shows that the composition for improving intestinal flora of the present invention has an effect of reducing fat accumulation of the liver and viscera.

### (Test example 6)

Twenty-one mice were divided into a normal diet group consisting of 7 mice, a high-cholesterol diet group consisting of 7 mice, and a test diet group consisting of 7 mice in which 0.1% of gnetin C separated and purified in Production example 2 was included in the high-cholesterol diet. The mice were fed ad libitum with these diets in an amount of 5g/mouse/day for 21 consecutive weeks and then their bloods were collected to measure the total cholesterol amount, the free cholesterol amount, triglyceride in the blood plasma using a biochemical analyzer. Further, the amount of alanine aminotransferase (ALT), indicative of liver function in the blood plasma, was measured by using Assay Kit Funakoshi K752-100.

Specifically, phosphate-buffered saline containing 3% fetal bovine serum was injected into mice and their bloods were collected from the abdomens using syringes with needles. The collected liquid was subjected to centrifugal separation under cooling (1000 rpm, 4°C, 5 min) to remove the supernatant, thereby obtaining macrophages. The total RNAs were extracted from these macrophages using an RNeasy mini kit (Qiagen K.K.). cDNAs were synthesized from the total RNAs using PrimeScript RT Master Mix (Takara Bio Inc.). Each cDNA (MCP-1, Col3α1, Cd11c) was subjected to quantitative RT-PCR measurement using SYBR Premix ExTaII (Takara Bio Inc.) and PikoReal (Themo Fisher Scientific K.K.).

After collecting the bloods, the mice were dissected to measure the liver weight and observe the liver under the microscope (Fig. 1). Note that the liver was subjected to Masson's trichrome staining to confirm fibrosis of the liver (fibrosis: stained parts). Further, the liver was subjected to Hematoxylin-Eosin staining to clearly confirm globules of fat (globules of fat: non-stained parts). The result is shown in Table 7.

**[Table 7]**

| Item | Normal diet group | High-fat diet group | Test diet group |
|---|---|---|---|
| Liver weight (g) | 0.94 | 1.42 | 0.8 |
| Total cholesterol (mg/dL) | 153 | 229 | 203 |
| Free cholesterol (mg/dL) | 34 | 54 | 39 |
| Triglyceride (mg/dL) | 54 | 30 | 15 |
| ALT (nM) | 0.037 | 0.302 | 0.17 |
| MCP-1 | 0.38 | 2.46 | 1.55 |
| C o 1 3 *α* 1 | 0.34 | 2.64 | 1.01 |
| C d 1 1 c | 0.29 | 1.98 | 0.97 |

| | | | |
|---|---|---|---|
| (n=7) | | | |

The liver weight, the total cholesterol amount, the free cholesterol amount, the amounts of triglyceride and ALT in the high-cholesterol diet group were 1.5 times, 1.5 times, 1.6 times, 0.6 times, and 8.2 times those in the normal diet group, respectively.

On the other hand, the liver weight, the total cholesterol amount, the free cholesterol amount, and the ALT amount in the test diet group were 0.9 times, 1.3 times, 1.1 times, 0.3 times, and 4.6 times those in the normal diet group, respectively.

In the high-cholesterol diet group, the blood cholesterol amount significantly increased, while, in the test diet group, the blood cholesterol amount was prevented from increasing and had the value close to that in the normal diet group.

Further, in the high-fat diet group, ALT significantly increased, while, in the test diet group, the ALT amount was about a half of that in the high-fat diet group.

This result shows that the composition for improving intestinal flora of the present invention has an effect of improving the liver function or preventing deterioration of the liver function.

The levels of MCP-1, Col3α1, Cd11c in the high-cholesterol diet group were 6.5 times, 7.8 times, and 6.8 times those in the normal diet group, respectively. The mice in the high-cholesterol diet group had a significant increase in all of the markers and exhibited nonalcoholic steatohepatitis (NASH) with progression of liver damages.

On the other hand, the levels of MCP-1, Col3α1, Cd11c in the test diet group were 4.1 times, 3.0 times, and 3.3 times those in the normal diet group, respectively.

This result shows that the composition for improving intestinal flora of the present invention can prevent deterioration of the liver function even if the high-cholesterol diet was ingested together.

When the liver tissues in the high-cholesterol diet group and the test diet group were observed under the microscope, fibrosis and large neutral lipid droplets were confirmed in the liver in the high-cholesterol diet group, while fibrosis was not observed and the size of the neutral lipid droplets was small in the test diet group (Fig. 1).

Further, the liver weight in the high-cholesterol diet group was 2.8 times that in the normal diet group, while the liver weight in the test diet group was 1.87 times that in the normal diet group. This result indicates that the composition for improving intestinal flora of the present invention has an effect of reducing the weight increase caused by the high-cholesterol diet.

These results show that the composition for improving intestinal flora of the present invention containing gnetin C as an active ingredient has effects of reducing the progression of and improving nonalcoholic steatohepatitis (NASH) and liver cancer caused by the weight increase due to fat accumulation in the liver and internal organs by ingestion of high-fat and high-cholesterol food, deterioration of the liver function, inflammation of the liver, liver fibrosis, or the like by improving the intestinal flora.

Further, it was confirmed, in Test example 3, that the amount of deoxycholic acid and the amount of lithocholic acid were significantly reduced by the intestinal flora improving effect in the test diet group. In this context, it has been known that farnesoid X receptor (FXR), which is a nuclear receptor having a bile acid as a ligand, is involved in cholesterol metabolism and lipid metabolism. Thus, it is speculated that the composition for improving intestinal flora of the present invention has an effect of reducing neutral fat accumulation in the liver by reducing the amounts of deoxycholic acid and lithocholic acid, which leads to a decrease in cholesterol metabolism and lipid metabolism, resulting in reduced fibrosis due to a drop in synthesis of ceramides.

### (Test example 7)

Seven mice were fed ad libitum with the test diet in which 0.5% of gnemonoside A separated and purified in Example 2 was included in the high-fat diet in an amount of 5g/mouse/day for 14 consecutive weeks, and then 10 mg of their excrement was collected. The excrement thus collected and 10 mg of the excrement collected in Test example 4 were each added with methanol (acetonitrile aqueous solution), suspended by an ultrasonic treatment, and then filtered. The filtrate was washed with hexane and passed through an ODS cartridge, and 20 µL of the elute was applied to an HPLC apparatus.

The HPLC chromatogram of the excrement collected in Test example 4 showed peaks of gnetin C and monoglucuronic acid conjugate of gnetin C.

On the other hand, the HPLC chromatogram of the excrement collected in Test example 7 showed peaks of gnetin C and the monoglucuronide, but not a peak of gnemonoside A, C, or D.

This result shows that gnemonoside A is hydrolyzed and changed to gnetin C in the intestinal tract.

Thus, it is found that the composition for improving intestinal flora of the present invention containing gnemonoside A, gnemonoside C, and gnemonoside D as active ingredients has the intestinal flora improving effect in the same manner as when it contains gnetin C as an active ingredient since these components are hydrolyzed into gnetin C in the intestinal tract.

### Production example 3 (production of tablet)

One part of the melinjo extract powders produced in Production example 1, 0.5 parts of a sucrose fatty acid ester, 0.5 parts of lactose, and 0.2 parts of crystalline cellulose were mixed and then the resulting mixture was compressed by a tablet press to produce a tablet having a diameter of 8 mm and a weight of 200 mg (gnemonoside A: 15 mg, gnemonoside D: 4.4 mg, gnetin C: 3.2 mg).

### Production example 4 (production of soft capsule agent)

One part of the gnetin C powders produced in Example 2, 1 part of a glycerol fatty acid ester, 0.2 parts of soybean lecithin, and 1.8 parts of corn oil were sufficiently mixed and then the resulting mixture was filled in a gelatin capsule to produce a soft capsule (contents of 200 mg, gnetin C: 50 mg).

### Production example 5 (production of cookie)

One part of gnemonoside A produced in Example 2, 2.5 parts of cake flour, 1 part of whole egg, 2 parts of butter, 1.5 parts of superfine sugar, 1.5 parts of butter, 0.3 parts of skim milk, 0.02 parts of baking powders, and 0.8 parts of water were mixed, kneaded, and then baked in an oven to produce a cookie (gnemonoside A: 16%).

### Industrial Applicability

The melinjo extract which uses the melinjo seed endosperms frequently eaten in Indonesia as a raw material is safe. Gnetin C and/or the glycoside thereof serving as the main components of the melinjo extract have the effect of increasing the abundance ratio of Akkermansia. Thus, the melinjo extract is useful not only for preventing and treating the liver function disease such as nonalcoholic fatty liver disease (NAFLD) or nonalcoholic steatohepatitis (NASH), liver cancer, colorectal cancer, inflammatory bowel disease, and the like by improving the intestinal flora, but also for treating cancer by improving the effect of the immune checkpoint inhibitor and for improving lifestyle disease by regulating the bile acid metabolism. The composition for improving intestinal flora of the present invention containing, as active ingredients, gnetin C and/or the glycoside thereof, which are the main components of the melinjo extract having such effects, can be prepared as a solid product and a powdery product by including a compounding agent for facilitating administration and ingestion and then easily processed into a preparation such as a granular agent, a tablet, a capsule agent, or a drinkable preparation, and food and drink, allowing the composition for improving intestinal flora of the present invention to contribute to improving symptoms of patients.

## Claims

1. Composition comprising gnetin C and/or its glycoside as an active ingredient for use in preventing and/or treating nonalcoholic steatohepatitis by improving intestinal flora.

2. Composition for use according to claim 1, wherein the glycoside is one or more selected from gnemonoside A, gnemonoside C, or gnemonoside D.

## Patentansprüche

1. Zusammensetzung, die Gnetin C und/oder sein Glycosid als Wirkstoff umfasst, zur Verwendung bei der Prävention und/oder Behandlung von nichtalkoholischer Steatohepatitis durch Verbesserung der Darmflora.

2. Zusammensetzung zur Verwendung gemäß Anspruch 1, wobei es sich bei dem Glycosid um eines oder mehrere handelt, die aus Gnemonosid A, Gnemonosid C oder Gnemonosid D ausgewählt sind.

## Revendications

1. Composition comprenant de la gnétine C et/ou son glycoside comme principe actif à utiliser dans la prévention et/ou le traitement d'une stéatohépatite non alcoolique en améliorant la flore intestinale.

2. Composition à utiliser selon la revendication 1, dans laquelle ledit glycoside est l'un ou plusieurs choisis parmi le gnémonoside A, le gnémonoside C, ou le gnémonoside D.
